(19) **Europäisches Patentamt / European Patent Office / Office européen des brevets**

(11) **EP 4 534 685 B1**

(12) **EUROPEAN PATENT SPECIFICATION**

(45) Date of publication and mention
of the grant of the patent:
**29.04.2026 Bulletin 2026/18**

(21) Application number: **22943151.5**

(22) Date of filing: **26.05.2022**

(51) International Patent Classification (IPC):
**G16B 20/20** (2019.01)   **C12Q 1/68** (2018.01)
**C12N 15/00** (2006.01)   C12Q 1/6827 (2018.01)

(52) Cooperative Patent Classification (CPC):
(C-Sets available)
**C12Q 1/6858; G16B 20/20;** G16B 20/10;
G16B 30/10                                    (Cont.)

(86) International application number:
**PCT/CN2022/095279**

(87) International publication number:
**WO 2023/225951 (30.11.2023 Gazette 2023/48)**

(54) **METHOD FOR DETECTING FETAL GENOTYPE ON BASIS OF HAPLOTYPE**

VERFAHREN ZUM NACHWEIS FÖTALER GENOTYPEN AUF DER BASIS VON HAPLOTYPEN

PROCÉDÉ DE DÉTECTION DU GÉNOTYPE FOETAL À PARTIR D'UN HAPLOTYPE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**09.04.2025 Bulletin 2025/15**

(73) Proprietor: **BGI Shenzhen**
**Shenzhen, Guangdong 518083 (CN)**

(72) Inventors:
• **JU, Jia**
**Shenzhen, Guangdong 518083 (CN)**
• **LI, Jia**
**Shenzhen, Guangdong 518083 (CN)**
• **SU, Fengxia**
**Shenzhen, Guangdong 518083 (CN)**
• **GAO, Ya**
**Shenzhen, Guangdong 518083 (CN)**
• **JIN, Xin**
**Shenzhen, Guangdong 518083 (CN)**
• **LIN, Yu**
**Shenzhen, Guangdong 518083 (CN)**

(74) Representative: **Meissner Bolte Nürnberg**
**Patentanwälte Rechtsanwälte**
**Partnerschaft mbB**
**Bankgasse 3**
**90402 Nürnberg (DE)**

(56) References cited:
WO-A1-2018/121468    WO-A1-2021/155831
CN-A- 101 346 724    CN-A- 104 053 789
CN-A- 112 840 404    US-A1- 2010 099 092
US-A1- 2021 340 601

• JUJIA-BIOINFORMATICS: "Non-invasive
prediction of fetal genotype using parental
haplotypes and the cell-free DNA sequencing of
maternal plasma", GITHUB, 27 April 2022
(2022-04-27), XP093290409, Retrieved from the
Internet <URL:https://github.com/jujia-BGI/
Prediction-of-fetal-genotype-using-a-haplotype-
based-method>
• JUJIA-BIOINFORMATICS: "Commits . jujia-BGI/
Prediction-of-fetal-genotype-using-a-haplotype-
based-method . GitHub", GITHUB, 27 April 2022
(2022-04-27), XP093290413, Retrieved from the
Internet <URL:https://github.com/jujia-BGI/
Prediction-of-fetal-genotype-using-a-haplotype-
based-method/commits/main/>

- JUJIA-BIOINFORMATICS: "Update README.md . jujia-BGI/Prediction-of-fetal-genotype-using-a-haplotype-based-method@1a3cac5 . GitHub", 27 April 2022 (2022-04-27), XP093290415, Retrieved from the Internet <URL:https://github.com/jujia-BGI/Prediction-of-fetal-genotype-using-a-haplotype-based-method/commit/1a3cac579a904cf5986998e2a678cf576fafc62c>
- KWAK SOO HEON, POWE CAMILLE E, JANG SE SONG, CALLAHAN MICHAEL J, BERNSTEIN SARAH N, LEE SEUNG MI, KANG SUNYOUNG, PARK KYONG SOO, J: "Sequencing Cell-free Fetal DNA in Pregnant Women With GCK -MODY", JOURNAL OF CLINICAL ENDOCRINOLOGY AND METABOLISM, THE ENDOCRINE SOCIETY, US, vol. 106, no. 9, 18 August 2021 (2021-08-18), US , pages 2678 - 2689, XP093111828, ISSN: 0021-972X, DOI: 10.1210/clinem/dgab265

(52) Cooperative Patent Classification (CPC): (Cont.)

C-Sets
**C12Q 1/6858, C12Q 2535/122, C12Q 2537/165**

## Description

### TECHNICAL FIELD

The present disclosure belongs to the field of biotechnology. More specifically, the present disclosure provides a haplotype-based method for detecting fetal genotype.

### BACKGROUND OF THE INVENTION

Since 2000, in the field of non-invasive prenatal detection for monogenic diseases, researches and clinical applications of cfDNA in the peripheral plasma of pregnant women by PCR and related technologies were aimed at providing a clear diagnosis of the monogenic disease (Saito, Sekizawa *et al.* 2000), such as myotonic dystrophy (Amicucci, Gennarelli *et al.* 2000). Such detection methods mainly target a single gene or variant, and detect paternally inherited mutations and fetal *de novo* mutations by determining the presence of foreign alleles in maternal peripheral blood that differ from the genotypes of mother.

For maternal heterozygous loci, due to the mixing of fetal and maternal DNA fragments in plasma of pregnant women, the interference of a large amount of maternal DNA information makes it difficult to determine whether a fetus carries maternal mutations and impossible to determine whether there is an allele different from the mother by qualitative analysis. Therefore, quantitative analysis is necessary for each type of allele. Digital PCR, the first method to infer maternally inherited mutations, is capable of detecting minor allele imbalances. The use of relative mutation dosage (RMD) analysis in determining the change of the proportion of the two alleles and its relationship with FF (Fetal Fraction, the concentration of cfDNA from fetus) on maternal heterozygous loci has been validated in different diseases (Tsui, Kadir *et al.* 2011, Barrett, McDonnell *et al.* 2012).

The above mentioned methods all focus on specific variants or a small gene region. With the development of next generation sequencing technology, it becomes possible to perform whole-exome sequencing (WES) or whole genome sequencing (WGS) of cfDNA in maternal plasma to infer fetal genome, monogenic variant and *do novo* mutation in whole genome, based on the presence of fetal cfDNA in plasma of pregnant women.

Lo *et al.* used high-depth sequencing of cfDNA to analyze the maternal haplotype, and developed a method for revealing the entire fetal genome based on relative haplotype dosage (RHDO), which lays a foundation for research in this field (Lo, Chan *et al.* 2010). The method uses the imbalance of read coverage of two alleles at heterozygous loci in plasma cfDNA sequencing data of pregnant women, to develop a relative haplotype dosage (RHDO) method to identify loci in the fetal genotype that are heterozygous in mother and homozygous in father.

In the years following the publication of Lo *et al.*'s work, there was a focus on improving haplotype-based methods. These researches focused on using various techniques to directly analyze maternal haplotype to avoid dependence on haplotypes of other family members such as a proband (Fan, Gu *et al.* 2012, Kitzman, Snyder *et al.* 2012). The method uses cfDNA data and single cell sequencing data of the plasma in pregnant women for fetal genome inference (Fan *et al.,* 2012). The method comprises: firstly, extracting lymphocytes from the peripheral blood of a pregnant woman to perform the single cell sequencing; obtaining the highly accurate haplotype at the level of maternal two chromosomes by using the direct deterministic phasing method; performing whole genome (or exome) sequencing of cfDNA in the plasma of the pregnant woman; determining the fetal haplotype inherited from the mother by counting the number of reads at maternal heterozygous loci in the plasma of the pregnant woman; then, determining the fetal allele inherited from the paternal heterozygote loci by counting the number of reads of paternally specific heterozygote loci in the plasma of the pregnant woman; using these paternal heterozygote loci to reconstruct the paternal haplotype, so as to infer the fetal haplotype inherited from the father; and finally, obtaining the fetal genome by combining these two haplotypes.

A common limitation of the above methods, which use cfDNA data in plasma of pregnant women and parental haplotype sequencing data to infer fetal genome, is that they rely on haplotype information. These methods either rely on the haplotype information of a proband of the family to reconstruct the maternal haplotype, or rely on using not widely used and complex experimental techniques to obtain the maternal haplotype, which requires a lot of professional knowledge and is time-consuming. Due to various reasons, these methods cannot type the entire fetal genome and only cover some of the genomic loci, and thus cannot complete the fetal genotype inference for the loci that cannot be covered by haplotype. The use of haplotype requires dealing with recombination events and genotyping errors. Some methods cannot determine the variants of which both the father and mother are heterozygous. These methods cannot detect *do novo* mutations and cannot provide reliable inferences for InDel genotype. The haplotype-based methods have many limitations, and cannot infer the genotypes at all fetal loci at early gestation due to the problems such as low FF and limitations of sequencing methods.

As the cost of high-throughput sequencing decreases, researchers have tried to use more reliable sequencing methods for high-depth sequencing, such as PCR-free WGS sequencing technology for cfDNA in plasma of pregnant women. Chan *et al.* used PCR-free experimental protocol to reduce sequencing errors and performed ultra-deep cfDNA WGS sequencing to develop a genome-wide relative allelic dosage (GRAD) analysis, which has led to two major frontiers in genome-wide NIPD for monogenic diseases: inference of maternal heterozygous loci and detection of *de novo* mutations (Chan, Jiang *et al.* 2016). The method uses ultra-deep sequencing (~270×) of cfDNA in plasma of pregnant women and parental whole-genome sequencing (WGS) data to infer the fetal genome. The method comprises: firstly, performing alignment and variant calling for maternal plasma cfDNA data and parental WGS data; using PCR-free experimental protocol to reduce sequencing errors and performing ultra-deep cfDNA WGS sequencing to develop a genome-wide relative allelic dosage (GRAD) analysis, so as to obtain the number of reads at each locus in maternal plasma, to further obtain the fetal genome. The advantages of the method are that parental haplotype is not necessary and *de novo* mutations can be inferred. The limitations of the method include: there is no inference for the loci where both the father and mother are heterozygous; high FF is required; and the number of covered maternal heterozygous loci is much smaller than the theoretical number. Next, Rabinowitz *et al.* used high-throughput sequencing technology to perform high-depth sequencing of cfDNA in plasma of pregnant women, which did not require the parental haplotype information, and used Bayesian model combined with machine learning methods to detect InDels and the loci where both the father and mother are heterozygous, with a lower overall detection accuracy compared to other algorithms (Rabinowitz, Polsky *et al.* 2019).

The use of ultra-deep sequencing data of cfDNA in plasma of pregnant women for inferring fetal genome genotype can achieve full coverage of fetal genomic loci. However, extremely high sequencing depth (above 300×) is required because a fixed threshold is used for each locus to determine fetal genotype. Fetal genotype cannot be accurately inferred when the sequencing depth is low (100×) or the fetal concentration is low.

References:

Amicucci, P., M. Gennarelli, G. Novelli and B. Dallapiccola (2000). "Prenatal Diagnosis of Myotonic Dystrophy Using Fetal DNA Obtained from Maternal Plasma." Clinical Chemistry 46(2): 301-302.

Barrett, A. N., T. C. McDonnell, K. C. Chan and L. S. Chitty (2012). "Digital PCR analysis of maternal plasma for noninvasive detection of sickle cell anemia." Clin Chem 58(6): 1026-1032.

Chan, K. C., P. Jiang, K. Sun, Y K. Cheng, Y. K. Tong, S. H. Cheng, A. I. Wong, I. Hudecova, T. Y. Leung, R. W. Chiu and Y. M. Lo (2016). "Second generation noninvasive fetal genome analysis reveals de novo mutations, single-base parental inheritance, and preferred DNA ends." Proc Natl Acad Sci U S A 113(50): E8159-E8168.

Fan, H. C., W. Gu, J. Wang, Y. J. Blumenfeld, Y. Y. El-Sayed and S. R. Quake (2012). "Non-invasive prenatal measurement of the fetal genome." Nature 487(7407): 320-324.

Kitzman, J. O., M. W. Snyder, M. Ventura, A. P. Lewis, R. Qiu, L. E. Simmons, H. S. Gammill, C. E. Rubens, D. A. Santillan, J. C. Murray, H. K. Tabor, M. J. Bamshad, E. E. Eichler and J. Shendure (2012). "Noninvasive whole-genome sequencing of a human fetus." Sci Transl Med 4(137): 137ra176.

Lo, Y. M., K. C. Chan, H. Sun, E.Z. Chen, P. Jiang, F. M. Lun, Y. W. Zheng, T. Y. Leung, T. K. Lau, C. R. Cantor and R. W. Chiu (2010). "Maternal plasma DNA sequencing reveals the genome-wide genetic and mutational profile of the fetus." Sci Transl Med 2(61): 61ra91.

Rabinowitz, T., A. Polsky, D. Golan, A. Danilevsky, G. Shapira, C. Raff, L. Basel-Salmon, R. T. Matar and N. Shomron (2019). "Bayesian-based noninvasive prenatal diagnosis of single-gene disorders." Genome Res 29(3): 428-438.

Saito, H., A. Sekizawa, T. Morimoto, M. Suzuki and T. Yanaihara (2000). "Prenatal DNA diagnosis of a single-gene disorder from maternal plasma." Lancet 356(9236): 1170.

Tsui, N. B. Y, R. A. Kadir, K. C. A. Chan, C. Chi and Y. M. D. Lo (2011). "Noninvasive prenatal diagnosis of hemophilia by microfluidics digital PCR analysis of maternal plasma DNA." Blood 117(13): 3684-3691.

## SUMMARY **OF** THE INVENTION

To solve at least some of the problems in the prior art, the present disclosure aims to provide a fetal genotype detection method for genome-wide single nucleotide variant detection, which provides an accurate result and effectively reduces sequencing depth.

Therefore, the present disclosure provides a haplotype-based method for detecting fetal genotype, the method comprising:

1) performing cfDNA sequencing on a plasma sample of a pregnant woman to be detected with known maternal and paternal haplotypes to obtain sequencing reads;

2) aligning the sequencing reads to a reference genome, and determining the depth distribution and mutations of the sample according to the alignment results;

3) calculating the fetal cfDNA concentration according to the minor allele frequency for four types of allelic loci, wherein the four types of allelic loci comprise: an allelic locus where the mother is homozygous and the fetus is homozygous, an allelic locus where the mother is homozygous and the fetus is heterozygous, an allelic locus where the mother is heterozygous and the fetus is homozygous, and an allelic locus where the mother is heterozygous and the fetus is heterozygous; and

4) for the parental genotype combinations AAAB, ABAA and ABAB, inferring fetal SNP and InDel genotypes as follows:

a) for the parental genotype combination AAAB, using Bayesian algorithm to infer the fetal SNP genotype, and using CIVA method to infer the fetal InDel alleles inherited from the father based on the SNP haplotype inherited from the father;

b) for the parental genotype combination ABAA, using SPRT algorithm to infer a locus that can be covered by the haplotype and using CIVA method to infer the remaining loci;

c) for the parental genotype combination ABAB, firstly using CIVA method to determine the fetal locus type inherited from the father; then, using SPRT algorithm to infer the fetal locus type inherited from the mother; for the locus that cannot be inferred by SPRT algorithm, using CIVA method to infer the fetal allele type inherited from the mother; combining the paternal allele and maternal allele, so as to obtain the fetal genotype of the locus;

d) for a locus that cannot be covered by the haplotype data in the above three genotype combinations, using Bayesian algorithm to determine the genotypes of the locus; and

e) in the case where the fetal locus inferred by Bayesian algorithm is different from the parental locus, a fetal *de novo* mutation is identified.

In one embodiment, in 4),

the Bayesian algorithm comprises: constructing a Bayesian model based on the fetal concentration to infer the fetal genotype;

the SPRT algorithm comprises: identifying the fetal genotype at a locus where the mother is heterozygous and the father is homozygous by using the imbalance of read coverage of two alleles at heterozygous loci in the plasma cfDNA sequencing data of the pregnant woman; and

the CIVA method comprises: inferring two haplotypes of two alleles inherited from the father and the mother on the fetal locus to be predicted, respectively, and extracting the alleles that are actually at the locus in the two haplotypes, so as to infer the fetal genotype.

In one embodiment, in 4), the Bayesian algorithm comprises: for each locus, when the read depth of different alleles and fetal concentration in the plasma of the pregnant woman are known, the probability of obtaining a combination i of maternal and fetal genotypes is calculated as follows:

$$P(A_i|B) = \frac{P(B|A_i)P(A_i)}{\sum_{i=1}^{n} P(B|A_i)P(A_i)}$$

wherein, $P(A_i)$ is a prior probability of the combination *i* in *n* combinations of maternal and fetal genotypes obtained based on the fetal concentration; $P(B|A_i)$ is the cumulative probability of the combination i of maternal and fetal genotypes calculated based on the read depth at the locus and the prior probability; n combinations of maternal and fetal genotypes = (10 maternal genotypes $\times$ 10 fetal genotypes); when the paternal and maternal genotypes are known, the combination in the calculation of the prior probability is modified for the above Bayesian algorithm, to remove the combinations of maternal and fetal genotypes that do not conform to Mendel's laws of heredity, and the probability value of each combination is recalculated; and finally, the combination with highest probability is selected, so as to obtain the fetal genotype.

In one embodiment, in the SPRT algorithm, all SNPs in the maternal genome are classified as $\alpha$ type and $\beta$ type, and two haplotypes of the mother are numbered as Hap I and Hap II; the $\alpha$ type SNP is defined as the allele of the father in the SNP being the same as that of the mother in Hap I, and the $\beta$ type SNP is defined as the allele of the father in the SNP being the same as that of the mother in Hap II; for the $\alpha$ type SNP, the number of allele reads at the locus in Hap I increases when the fetus inherits Hap I from the mother; however, the numbers of reads of two alleles at the locus are close to balance when the fetus inherits Hap II from the mother; for the $\beta$ type SNP, the numbers of reads of two alleles at the locus are close to balance when the fetus inherits Hap I from the mother; however, the number of allele reads at the locus in Hap II increases when the fetus inherits Hap II from the mother.

In one embodiment, in the SPRT algorithm, the following two thresholds are used to determine whether two alleles at each locus are balanced,

$$upper\ boundary = \frac{(ln\ 1200)/N - ln\ d}{ln\ g}$$

and

$$lower\ boundary = \frac{(ln\ 1/1200)/N - ln\ d}{ln\ g}$$

wherein,

$$d = \frac{1 - q_1}{1 - q_0}$$

and

$$g = \frac{q_1(1 - q_0)}{q_0(1 - q_1)}$$

wherein, $q_0$ is the number of reads of Hap I at the locus when the fetus inherits Hap II from mother, $q_1$ is the number of reads of Hap I at the locus when the fetus inherits Hap I from mother, and N is the total number of reads; for the $\alpha$ type SNP, $q_0$=0.5, $q_1$=ff/2+0.5; for the $\beta$ type SNP, $q_1$=0.5, $q_0$=0.5-ff/2.

In one embodiment, in e) of 4), *a de novo* mutation in the fetus is further screened by the following criteria:

i) all variants recorded in dbSNP147, Genome Aggregation Database (gnomAD) with minor allele frequency (MAF) > 1% in East Asian population, and internal database;
ii) a locus with the paternal or maternal or fetal genotype quality (GQ) < 30;
iii) a locus with the paternal or maternal or fetal sequencing depth < 20;
iv) a locus with the paternal or maternal or fetal genotype likelihood (PL) < 30;
v) a locus with the paternal or maternal or fetal minor allele frequency < 0.3 or > 0.7; and
vi) a locus with the following quality control criteria in the combined VCF (Variant Call Format, generated by GATK) of parent and fetus: QualByDepth (QD) < 2.0, RMSMappingQuality (MQ) < 50.0, FisherStrand (FS) > 60.0, StrandOddsRatio (SOR) > 3.0, MappingQualityRankSumTest (MQRankSum) < -12.5, ReadPosRankSumTest (ReadPosRankSum) < -8.0.

In one embodiment, in 1), the maternal and paternal haplotypes are obtained by stLFR (single tube Long Fragment Read) sequencing of the maternal and paternal blood cells, respectively.
In one embodiment, in 1), the depth of cfDNA sequencing is 70-200× (e.g., 100×).
The embodiments of the present disclosure extend the scope of fetal genotype inference to single nucleotide variant of the whole genome by using the whole genome sequencing data of free DNA in the plasma of pregnant women, which provides support for extending the scope of fetal genetic disease screening. It further improves the accuracy of fetal genotype inference using the plasma of pregnant women alone by combining haplotype algorithm with Bayesian model. The accuracy is still high at a fetal cfDNA concentration of 4% and a sequencing depth of free DNA in the plasma of pregnant women of 100×. The scope of fetal genotype inference is extended to InDels.

## BRIEF DESCRIPTION OF THE DRAWINGS

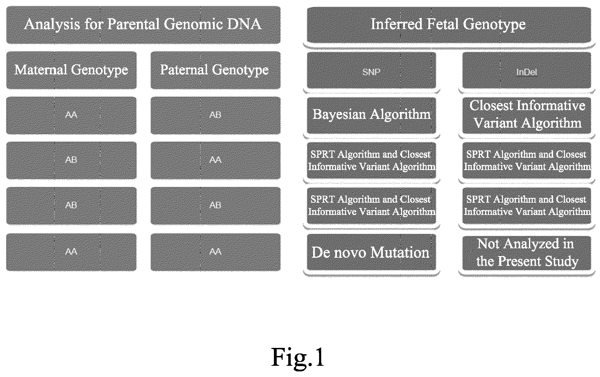

Figure 1 shows a schematic diagram of the method for inferring fetal genotype.

## DETAILED DESCRIPTION OF THE INVENTION

The embodiments of the present disclosure can accurately infer the fetal genotype by high-depth whole genome sequencing of free DNA in the peripheral blood of pregnant women and stLFR sequencing of the parental blood cells, so as to screen for abnormal conditions in the fetal genome. In the present disclosure, stLFR sequencing can be used to obtain parental haplotype information, and the accuracy of inferred paternal and maternal haplotypes can be further improved by introducing a Hidden Markov Model or methods based on fragment assembly, so as to improve the overall accuracy. In the present disclosure, other haplotype sequencing methods can be used instead of stLFR sequencing. The present disclosure is of great significance for detecting pathogenic loci of a variety of genetic diseases, such as monogenic diseases, polygenic diseases and chromosomal diseases, which may be carried by fetus. Further, the embodiments of the present disclosure can be used in the fields such as disease inference during pregnancy, fetal phenotype inference, etc.

The embodiments of the present disclosure: do not depend on the haplotype information of proband; extend the scope of fetal genome analysis to the whole genome; improve the detection accuracy to a single base mutation; expand the application of fetal genetic disease detection, including the loci where mother is homozygous and father is heterozygous, the loci where mother is heterozygous and father is homozygous, and the loci where both mother and father are heterozygous; detect variant types comprising SNP, InDel and fetal *de novo* mutations; do not depend on the fetal concentration, with high precision when the fetal concentration is low (4%); and can achieve high accuracy when the sequencing depth is relatively low (~100×).

The embodiments of the present disclosure are explained in accompanying with an exemplary embodiment as below. In this embodiment, the haplotype-based method for detecting fetal genotype comprises:

1) performing high-depth sequencing (e.g., 100×) of cfDNA in the plasma of a pregnant woman and stLFR sequencing of maternal and fetal paternal blood cells for each family to be analyzed;

2) aligning all raw data (fq format) after quality control to the human reference genome by using alignment software such as BWA (mem mode); using methods such as Picard to remove the duplicate reads in the alignment and calculating the duplication rate; using the Base Quality Score Recalibration (BQSR) function in variant calling algorithms such as GATK to conduct the local correction in the alignment; using the Depth of Coverage function in variant calling algorithms such as GATK to calculate the depth distribution of each sample; using the population variant calling mode in variant calling algorithms such as GATK to detect single nucleotide polymorphisms (SNPs) and insertions/deletions (Indels) of small fragments;

3) using tools such as Hapcut2 or Longhap to assemble long fragments and analyze the sample haplotype based on barcode information for the sequencing data of single-tube long fragment reads (stLFRs) of paternal and maternal blood cells;

4) calculating fetal cfDNA concentration: calculating the distribution of minor allele frequency (MAF), which is expected to have four peaks corresponding to four types of loci: a locus where the mother is homozygous and the fetus is homozygous, a locus where the mother is homozygous and the fetus is heterozygous, a locus where the mother is heterozygous and the fetus is homozygous, and a locus where the mother is heterozygous and the fetus is heterozygous; for each locus, when the fetal concentration is f, MAF corresponding to the peak of the locus where the mother is homozygous and the fetus is heterozygous is f/2 given that MAF distribution is obtained from reads of pregnant women and fetuses; therefore, for MAF $\in$ [0,0.25], if the distribution is simulated as a mixed model of two normal distributions, it can be inferred that MAF corresponding to the peak in the read distribution obtained from the fetus is f/2, and the fetal concentration in the sample can be obtained;

5) Bayesian algorithm: establishing a Bayesian model based on the fetal cfDNA concentration to infer the fetal genotype; for each locus, when the read depth of different alleles and fetal cfDNA concentration in the plasma of the pregnant woman are known, the probability of obtaining a combination i of maternal and fetal genotypes is calculated as follows:

$$P(A_i|B) = \frac{P(B|A_i)P(A_i)}{\sum_{i=1}^{n} P(B|A_i)P(A_i)}$$

wherein, $A_i$ is the fetal genotype of the combination *i* in *n* combinations, and B is the number of supported bases in the alleles observed in the data; $P(A_i)$ is the prior probability of the combination *i* in *n* combinations of maternal and fetal genotypes obtained based on the fetal concentration; $P(B|A_i)$ is the cumulative probability of the combination i of maternal and fetal genotypes calculated based on the read depth at the locus and the prior probability; n combinations of maternal and fetal genotypes = (10 maternal genotypes × 10 fetal genotypes); when the paternal and maternal genotypes are known, the combination in the calculation of the prior probability is modified for the above Bayesian model, to remove the combinations of maternal and fetal genotypes that do not conform to Mendel's laws of heredity, and the probability value of each combination is recalculated; and finally, the combination with highest probability is selected, so as to obtain the fetal genotype;

6) haplotype method a (SPRT (sequential probability ratio test) algorithm (Lo, Chan *et al.* 2010)): using the imbalance of read coverage of two alleles at heterozygous loci in the plasma cfDNA sequencing data of pregnant women to identify the fetal genotype at the loci where the mother is heterozygous and the father is homozygous.

Specifically, all single nucleotide polymorphisms (SNPs) in the maternal genome are classified as $\alpha$ type and $\beta$ type, and two haplotypes of the mother are numbered as Hap I and Hap II; the $\alpha$ type SNP is defined as the allele of the father in the SNP being the same as that of the mother in Hap I, and the $\beta$ type SNP is defined as the allele of the father in the SNP being the same as that of the mother in Hap II; for the $\alpha$ type SNP, the number of allele reads at the locus in Hap I increases when the fetus inherits Hap I from the mother; however, the numbers of reads of two alleles at the locus are close to balance when the fetus inherits Hap II from the mother; for the $\beta$ type SNP, the numbers of reads of two alleles at the locus are close to balance when the fetus inherits Hap I from the mother; however, the number of allele reads at the locus in Hap II increases when the fetus inherits Hap II from the mother.

Based on the above theory, two thresholds are introduced to determine whether two alleles at each locus are balanced. The two thresholds are calculated as follows:

$$upper\ boundary = \frac{(ln\ 1200)/N - ln\,d}{ln\,g}$$

and

$$lower\ boundary = \frac{(ln\ 1/1200)/N - ln\,d}{ln\,g}$$

wherein,

$$d = \frac{1 - q_1}{1 - q_0}$$

and

$$g = \frac{q_1(1 - q_0)}{q_0(1 - q_1)}$$

wherein, $q_0$ is the number of reads of Hap I at the locus when the fetus inherits Hap II from mother, $q_1$ is the number of reads of Hap I at the locus when the fetus inherits Hap I from mother, and N is the total number of reads;

for the $\alpha$ type SNP, $q_0$=0.5, $q_1$=ff/2+0.5; for the $\beta$ type SNP, $q_1$=0.5, $q_0$=0.5-ff/2.

The fetal alleles inherited from the father and the mother can be inferred by comparing the cumulative proportion of reads of the alleles that are actually inherited from the maternal Hap I and Hap II at each locus with the above two thresholds, and thus the fetal genotype can be determined. If the proportion of cumulative depth of alleles in the maternal Hap I to the total cumulative sequencing depth of two haplotypes is less than the lower boundary, the fetus inherits the maternal Hap II; if the proportion is greater than the upper boundary, the fetus inherits the maternal Hap I.

7) haplotype method b (closest informative variant algorithm, CIVA): inferring two haplotypes of two alleles inherited from the father and the mother on the fetal locus to be predicted, respectively, and extracting the alleles that are actually at the locus in the two haplotypes, so as to infer the fetal genotype;

Specifically, according to the linkage disequilibrium, the genetic information of the haplotype at the locus can be inferred by using the fetal genotype information inferred by the Bayesian algorithm or SPRT algorithm in the previous step and the fetal haplotype information inherited from the father or the mother of the nearest valuable variant locus within a certain region upstream and downstream of the analyzed locus. The length of a certain region can be 50 kb, 100 kb, 200 kb, etc.

In addition, a more rigorous method of determining whether a valuable variant locus is credible or not can be added. When two valuable variant loci in a certain region upstream and downstream of the analyzed locus indicate inconsistent haplotypes of the fetus inherited from the father or the mother, it is considered not credible, and CIVA cannot be used in this case.

There are valuable variant loci in the following two circumstances:

The locus where the mother is homozygous and the father is heterozygous is selected in the case of inferring haplotype from the father. If the father's genotype is AB, the mother's genotype is AA, and the fetus's genotype is AB, the fetus inherits the haplotype on which B allele is located from the father. If the father's genotype is AB, the mother's genotype is AA, and the fetus's genotype is AA, the fetus inherits the haplotype on which A allele is located from the father.

Similarly, the locus where the mother is heterozygous and the father is homozygous is used to infer haplotype from the mother. If the mother' genotype is AB, the father' genotype is BB, and the fetus' genotype is BB, the fetus inherits the haplotype on which B allele is located from the mother. If the mother' genotype is AB, the father' genotype is BB, and the fetus' genotype is AB, the fetus inherits the haplotype on which A allele is located from the mother.

8) the combined method of haplotype and Bayesian algorithm: combining the above Bayesian algorithm, SPRT algorithm based on the haplotype information and closest informative variant algorithm to infer the fetal genotype in terms of SNPs and InDels, wherein SNPs or InDels are classified into four types and the parental genotype combinations are AAAB, ABAA, ABAB and AAAA (Figure 1);
SNPs are inferred as follows:

a) for a SNP of AAAB, using Bayesian algorithm;
b) for a SNP of ABAA, using SPRT algorithm to infer a locus that can be covered by the haplotype and using CIVA method to infer the remaining loci that cannot be inferred by SPRT (the calculated value being between the two thresholds mentioned in the above SPRT method);
c) for a SNP of ABAB, using CIVA method to determine the fetal locus type inherited from the father; then, using SPRT algorithm to infer the fetal locus type inherited from the mother; for the locus that cannot be inferred by SPRT algorithm, using CIVA method to infer the fetal allele type inherited from the mother; combining the paternal allele and maternal allele to obtain the fetal genotype at the locus;
d) using Bayesian algorithm for the locus that cannot be covered by the haplotype data in the above three types of SNPs.

InDel variants are inferred as follows:

a) for an InDel of AAAB, using CIVA method to infer the fetal InDel alleles inherited from the father based on the haplotype (the SNP of AAAB) inherited from the father inferred in the previous step;
b) for an InDel of ABAA, using SPRT algorithm to infer the locus that can be covered by the haplotype and using CIVA method to infer the remaining loci that cannot be inferred by SPRT (the calculated value being between the two thresholds mentioned in the above SPRT method);
c) for an InDel of ABAB, firstly using CIVA method to determine the fetal locus type inherited from the father; then, using SPRT algorithm to infer the fetal locus type inherited from the mother; for the locus that cannot be inferred by SPRT algorithm, using CIVA method to infer the fetal allele type inherited from the mother; combining the paternal allele and maternal allele to obtain the fetal genotype at the locus;
d) using Bayesian algorithm for the locus that cannot be covered by the haplotype data in the above three types of InDels.

9) *de novo* SNP mutations in the fetus: when the fetal variant loci inferred by Bayesian algorithm are different from those of the father and the mother, *de novo* mutations in the fetus are screened by the following criteria:

i) all variants recorded in dbSNP147, Genome Aggregation Database (gnomAD) with minor allele frequency (MAF) > 1% in East Asian population, and internal database;
ii) a locus with the paternal or maternal or fetal genotype quality (GQ) < 30;
iii) a locus with the paternal or maternal or fetal sequencing depth < 20;
iv) a locus with the paternal or maternal or fetal genotype likelihood (PL) < 30;
v) a locus with the paternal or maternal or fetal minor allele frequency < 0.3 or > 0.7; and
vi) a locus with the following quality control criteria in the combined VCF of parent and fetus: QualByDepth (QD) < 2.0, RMSMappingQuality (MQ) < 50.0, FisherStrand (FS) > 60.0, StrandOddsRatio (SOR) > 3.0, MappingQualityRankSumTest (MQRankSum) < -12.5, ReadPosRankSumTest (ReadPosRankSum) < -8.0.

QD: QualByDepth, the quality value corrected by depth.
MQ: RMSMappingQuality, root mean square of the mapping quality of all reads, used to determine the average mapping quality at a locus.
FS: FisherStrand. Fisher's exact test is used to determine whether there is strand-specific bias for the current variant.
MQRankSum: MappingQualityRankSumTest, Mann-Whitney-Wilcoxon Rank Sum Test result for the mapping qualities among different bases at heterozygous loci, wherein the confidences of loci are evaluated based on the mapping qualities of REF versus ALT bases.
SOR: StrandOddsRatio, wherein OR value is used to determine the degree of strand bias at variant loci, and a greater value indicates a higher degree of strand bias.
ReadPosRankSum: ReadPosRankSumTest, Rank Sum Test for the heterozygous loci, used to determine whether different bases tend to appear at specific positions on the reads (for example, near the start or the end of reads).

The method of the present disclosure combines two haplotype algorithms with a Bayesian model to infer fetal single nucleotide variant based on the depth information in the plasma of pregnant women and parental haplotype information, which achieves full coverage of genomic loci. In the present disclosure, Bayesian algorithm can be further combined with the difference of cfDNA fragments between mother and fetus, such as fragment length, Motif type at the end of fragment,the difference of mapping positions on the genome to increase the weight of the fragments from fetus and improve the accuracy of Bayesian algorithm. For an ABAB locus, Bayesian algorithm is firstly used to determine the fetal haplotype inherited from father at the AAAB locus of the genome, then CIVA algorithm is used to determine the fetal haplotype inherited from the father at the ABAB locus, SPRT algorithm is used to predict the fetal haplotype inherited from the mother at the ABAB locus, and finally, the fetal ABAB genotype is inferred.

Examples

The present inventors had conducted sample collection, sequencing and data analysis of 50 healthy families and 9 patient families in 2021 and completed the fetal genome inference based on these data, so as to test the feasibility and accuracy of the present fetal genome inference method and further clarify its application scope.

The present disclosure conducted the tests on the maternal plasma cfDNA sequencing data (WGS paired-end 100 bp sequencing) and the paternal and maternal stLFR data (WGS paired-end 100 bp sequencing) of 5 families, and used the fetal cord blood WGS data (paired-end 100 bp) as a true positive set to calculate the accuracy of the fetal genotype inference (Tables 1, 2 and 3).

The inference accuracy of AAAB type variants in SNP loci of 5 families was 96.2%±5.8% (mean ± standard deviation), 96.2%±1.4% for ABAA and 87.2%±4.7% for ABAB.

The inference accuracy (mean ± standard deviation) of AAAB type variants in InDel loci of 5 families was 94.6% ±1.9%, 80.2%±4.3% for ABAA and 79.3%±3.3% for ABAB.

When the fetal concentration was 4% and the cfDNA sequencing depth was 128× (JK-53), the inference accuracies of SNPs were 95.6% (AAAB), 92% (ABAA) and 74.7% (ABAB), respectively.

When the fetal concentration was 4% and the cfDNA sequencing depth was 270× (JK-53), the inference for fetal de novo mutation had a sensitivity of 91.3% and a positive predictive value (PPV) of 53.9%.

Table 1. Statistics of sequencing data of test families

| Family ID | Sequencing Method | Number of Raw Reads | Number of Aligned Reads | Alignment Rate | Average Depth | Coverage Rate | Duplication Rate | N50 | Score of Phasing SNP |
|---|---|---|---|---|---|---|---|---|---|
| JK-7 | Cord Blood Cells WGS | 1233682194 | 1231279450 | 99.81% | 41.85 | 99.16% | 3.75% | | |
| JK-7 | Maternal efDNA WGS | 4930998263 | 4899353438 | 99.36% | 167.1 | 99.27% | 32.58% | | |
| JK-7 | Maternal Blood Cells StLFR | 2246895792 | 2213288894 | 98.50% | 68.39 | 99.32% | 53.12% | 38692373 | 99.8% |
| JK-7 | Paternal Blood Cells StLFR | 1847735151 | 1825890887 | 98.82% | 57.29 | 99.87% | 46.48% | 13595319 | 99.88% |
| JK-16 | Cord Blood Cells WGS | 1547702979 | 1544031563 | 99.76% | 52.34 | 99.16% | 3.15% | | |
| JK-16 | Maternal cfDNA WGS | 4470433381 | 4427313912 | 99.04% | 150.99 | 99.27% | 31.17% | | |
| JK-16 | Maternal Blood Cells StLFR | 1126578266 | 1093285410 | 97.04% | 34.05 | 99.20% | 37.45% | 43167036 | 99.78% |
| JK-16 | Paternal Blood Cells StLFR | 1071372133 | 1036265674 | 96.72% | 32.43 | 99.81% | 36.28% | 43812809 | 99.84% |
| JK-18 | Cord Blood Cells WGS | 1393244829 | 1391262033 | 99.86% | 47.27 | 99.14% | 3.49% | | |
| JK-18 | Maternal cfDNA WGS | 3410469123 | 3388616846 | 99.36% | 115.68 | 99.26% | 22.39% | | |
| JK-18 | Maternal Blood Cells StLFR | 1437373627 | 1404317757 | 97.70% | 42.84 | 99.27% | 36.91% | 7009682 | 99.76% |
| JK-18 | Paternal Blood Cells StLFR | 1419559729 | 1363089772 | 96.02% | 40.42 | 99.88% | 43.98% | 4303673 | 99.12% |
| JK-28 | Cord Blood Cells WGS | 1388613164 | 1386761047 | 99.87% | 47.38 | 99.13% | 4.18% | | |
| JK-28 | Maternal cfDNA WGS | 3311517554 | 3286161812 | 99.33% | 112.03 | 99.27% | 21.11% | | |
| JK-28 | Maternal Blood Cells StLFR | 1444610729 | 1412560944 | 97.78% | 42.08 | 99.27% | 38.33% | 1178766 | 99.05% |
| JK-28 | Paternal Blood Cells StLFR | 1284525331 | 1246303246 | 97.02% | 37.82 | 99.85% | 42.77% | 1424125 | 99.26% |
| JK-53 | Cord Blood Cells WGS | 1480341904 | 1477844272 | 99.83% | 50.33 | 99.13% | 4.23% | | |
| JK-53 | Maternal Plasma cfDNA | 8909756646 | 8870299941 | 99.56% | 256.12 | 99.29% | 40.84% | | |
| JK-53 | Maternal Blood Cells StLFR | 93.1259760 | 919002827 | 98.47% | 28.77 | 99.12% | 17.24% | 3352388 | 99.66% |
| JK-53 | Paternal Blood Cells StLFR | 1055522782 | 1035088382 | 98.06% | 31.51 | 99.76% | 13.31% | 4708438 | 99.64% |

Table 2. Summary of accuracy of SNP loci on the fetal genome inferred by different embodiments

| Family | FF | Heterozygote | Bayesian Model | | Haplotype-Based Method | | Combined Method | |
|---|---|---|---|---|---|---|---|---|
| | | | Accuracy | Number of True Positive Number of Loci | Accuracy | Number of True Positive/Number of Loci | Accuracy | Number of True Positive/Number o f Loci |
| JK-7 | 0.09 | AAAB | 96.8% | 6317881652391 | | | | |
| | | ABAA | 72.6% | 909990/1253908 | 98.8% | 1171962/1186610 | 96.1% | 1209126/1253908 |
| | | ABAB | 61.4% | 429479/698962 | 96.4% | 572296/593618 | 89.8% | 627424/698962 |
| JK-16 | 0.27 | AAAB | 97.7% | 576306/590073 | | | | |
| | | ABAA | 91.8% | 1014224/1104405 | 99.2% | 1061980/1070070 | 98% | 1082868/1104405 |
| | | ABAB | 85.9% | 540677/629324 | 97.5% | 562304/576574 | 94.9% | 597082/629324 |
| JK-18 | 0.14 | AAAB | 96.4% | 463476/480708 | | | | |
| | | ABAA | 75.7% | 901292/1190790 | 99% | 1112107/1123516 | 96.7% | 1151107/1190790 |
| | | ABAB | 65.9% | 439900/667062 | 92.7% | 488305/526841 | 86.5% | 576938/667062 |
| JK-28 | 0.12 | AAAB | 95.2% | 313500/329369 | | | | |
| | | ABAA | 69.5% | 440760/633774 | 98.5% | 581675/590448 | 96% | 608483/633774 |
| | | ABAB | 57.3% | 222910/388712 | 93.1% | 268695/288587 | 83.2% | 323556/388712 |
| JK-53 (128X) | 0.04 | AAAB | 95.6% | 540046/564870 | | | | |
| | | ABAA | 59.6% | 667583/1120831 | 97.3% | 926919/952660 | 92.0% | 998262/1085313 |
| | | ABAB | 49.4% | 325416/658784 | 89.1% | 363047/407606 | 74.7% | 481240/644645 |
| JK-53 (256.12X) | 0.04 | AAAB | 95% | 590368/621392 | | | | |
| | | ABAA | 63.5% | 706502/1112899 | 97.7% | 979699/1002260 | 93.6% | 1041875/1112899 |
| | | ABAB | 51% | 341350/668854 | 93.9% | 462865/492889 | 81.8% | 547115/668854 |

Table 3. Summary of accuracy of InDel loci on the fetal genome inferred by different embodiments

| | Number True Positive/Total Number of AAAB Loci | AAAB Accuracy | Number of True Positive/Total Number of ABAA Loci | ABAA Accuracy | Number of True Positive/Total Number of ABAB Loci | ABAB Accuracy | FF | Total Number of InDels | InDelswith SPRT Results | Propertion of InDels with SPRT Results |
|---|---|---|---|---|---|---|---|---|---|---|
| JK-7 | 95446/123935 | 77% | 190093/199209 | 95.4% | 95069/123443 | 77% | 0.09 | 460212 | 446587 | 97% |
| JK-16 | 73714/95314 | 77.3% | 142989/147016 | 97.3% | 74754/93231 | 80.2% | 0.27 | 358513 | 335561 | 93.6% |
| JK-18 | 82721/104098 | 79.5% | 158053/170435 | 92.7% | 84550/106000 | 79.8% | 0.14 | 402516 | 380533 | 94.5% |
| JK-28 | 37904/42794 | 88.6% | 71967/75357 | 95.5% | 40697/48082 | 84.6% | 0.12 | 174955 | 166233 | 95% |
| JK-53 | 70971/90470 | 78.4% | 131633/142619 | 92.3% | 69269/92726 | 74.7% | 0.04 | 342825 | 325815 | 95% |

**Claims**

1. A haplotype-based method for detecting fetal genotype, the method comprising:

   1) performing cfDNA sequencing on a plasma sample of a pregnant woman to be detected with known maternal and paternal haplotypes to obtain sequencing reads;
   2) aligning the sequencing reads to a reference genome, and determining the depth distribution and mutations of the sample according to the alignment results;
   3) calculating the fetal cfDNA concentration according to the minor allele frequency for four types of allelic loci, wherein the four types of allelic loci comprise: an allelic locus where the mother is homozygous and the fetus is homozygous, an allelic locus where the mother is homozygous and the fetus is heterozygous, an allelic locus where the mother is heterozygous and the fetus is homozygous, and an allelic locus where the mother is heterozygous and the fetus is heterozygous; and
   4) for the parental genotype combinations AAAB, ABAA and ABAB, inferring fetal SNP and InDel genotypes as follows:

      a) for the parental genotype combination AAAB, using Bayesian algorithm to infer the fetal SNP genotype, and using CIVA method to infer the fetal InDel alleles inherited from the father based on the SNP haplotype inherited from the father;
      b) for the parental genotype combination ABAA, using SPRT algorithm to infer a locus that can be covered by the haplotype and using CIVA method to infer the remaining loci;
      c) for the parental genotype combination ABAB, firstly using CIVA method to determine the fetal locus type inherited from the father; then, using SPRT algorithm to infer the fetal locus type inherited from the mother; for the locus that cannot be inferred by SPRT algorithm, using CIVA method to infer the fetal allele type inherited from the mother; combining the paternal allele and maternal allele, so as to obtain the fetal genotype at the locus;
      d) for a locus that cannot be covered by the haplotype data in the above three genotype combinations, using Bayesian algorithm to determine the genotypes of the locus; and
      e) in the case where the fetal locus inferred by Bayesian algorithm is different from the parental locus, a fetal *de novo* mutation is identified.

2. The method of claim 1, wherein in 4),

   the Bayesian algorithm comprises: constructing a Bayesian model based on the fetal concentration to infer the fetal genotype;
   the SPRT algorithm comprises: identifying the fetal genotype at a locus where the mother is heterozygous and the father is homozygous by using the imbalance of read coverage of two alleles at heterozygous loci in the plasma cfDNA sequencing data of the pregnant woman; and
   the CIVA method comprises: inferring two haplotypes of two alleles inherited from the father and the mother on the fetal locus to be predicted, respectively, and extracting the alleles that are actually at the locus in the two haplotypes, so as to infer the fetal genotype.

3. The method of claim 1 or 2, wherein in 4), the Bayesian algorithm comprises: for each locus, when the read depth of different alleles and fetal concentration in the plasma of the pregnant woman are known, the probability of obtaining a combination i of maternal and fetal genotypes is calculated as follows:

$$P(A_i|B) = \frac{P(B|A_i)P(A_i)}{\sum_{i=1}^{n} P(B|A_i)P(A_i)}$$

   wherein, $P(A_i)$ is a prior probability of the combination *i* in *n* combinations of maternal and fetal genotypes obtained based on the fetal concentration; $P(B|A_i)$ is the cumulative probability of the combination i of maternal and fetal genotypes calculated based on the read depth at the locus and the prior probability; n combinations of maternal and fetal genotypes = (10 maternal genotypes × 10 fetal genotypes); when the paternal and maternal genotypes are known, the combination in the calculation of the prior probability is modified for the above Bayesian algorithm, to remove the combinations of maternal and fetal genotypes that do not conform to Mendel's laws of heredity, and the probability value of each combination is recalculated; and finally, the combination with highest probability is selected, so as to obtain the fetal genotype.

4. The method of claim 1 or 2, wherein in the SPRT algorithm, all SNPs in the maternal genome are classified as $\alpha$ type and $\beta$ type, and two haplotypes of the mother are numbered as Hap I and Hap II; the $\alpha$ type SNP is defined as the allele of the father in the SNP being the same as that of the mother in Hap I, and the $\beta$ type SNP is defined as the allele of the father in the SNP being the same as that of the mother in Hap II; for the $\alpha$ type SNP, the number of allele reads at the locus in Hap I increases when the fetus inherits Hap I from the mother; however, the numbers of reads of two alleles at the locus are close to balance when the fetus inherits Hap II from the mother; for the $\beta$ type SNP, the numbers of reads of two alleles at the locus are close to balance when the fetus inherits Hap I from the mother; however, the number of allele reads at the locus in Hap II increases when the fetus inherits Hap II from the mother.

5. The method of claim 4, wherein in the SPRT algorithm, the following two thresholds are used to determine whether two alleles at each locus are balanced,

$$upper\ boundary = \frac{(ln\ 1200)/N\ - ln\ d}{ln\ g}$$

and

$$lower\ boundary = \frac{(ln\ 1/1200)/N\ - ln\ d}{ln\ g}$$

wherein,

$$d = \frac{1 - q_1}{1 - q_0}$$

and

$$g = \frac{q_1(1 - q_0)}{q_0(1 - q_1)}$$

wherein, $q_0$ is the number of reads of Hap I at the locus when the fetus inherits Hap II from mother, $q_1$ is the number of reads of Hap I at the locus when the fetus inherits Hap I from mother, and N is the total number of reads; for the $\alpha$ type SNP, $q_0$=0.5, $q_1$=ff/2+0.5; for the $\beta$ type SNP, $q_1$=0.5, $q_0$=0.5-ff/2.

6. The method of claim 1 or 2, wherein in e) of 4), a *de novo* mutation in the fetus is further screened by the following criteria:

i) all variants recorded in dbSNP147, Genome Aggregation Database (gnomAD) with minor allele frequency (MAF) > 1% in East Asian population, and internal database;
ii) a locus with the paternal or maternal or fetal genotype quality (GQ) < 30;
iii) a locus with the paternal or maternal or fetal sequencing depth < 20;
iv) a locus with the paternal or maternal or fetal genotype likelihood (PL) < 30;
v) a locus with the paternal or maternal or fetal minor allele frequency < 0.3 or > 0.7; and
vi) a locus with the following quality control criteria in the combined VCF (Variant Call Format, generated by GATK) of parent and fetus: QualByDepth (QD) < 2.0, RMSMappingQuality (MQ) < 50.0, FisherStrand (FS) > 60.0, StrandOddsRatio (SOR) > 3.0, MappingQualityRankSumTest(MQRankSum) <-12.5, ReadPosRankSumTest (ReadPosRankSum) < -8.0.

7. The method of claim 1 or 2, wherein in

1), the maternal and paternal haplotypes are obtained by stLFR

(single tube Long Fragment Read) sequencing of the maternal and paternal blood cells, respectively.

8. The method of claim 1 or 2, wherein in

   1), the depth of cfDNA sequencing is 70-200×.

9. The method of claim 8, wherein the depth of cfDNA sequencing is 100×.

**Patentansprüche**

1. Haplotypbasiertes Verfahren zum Ermitteln des fetalen Genotyps, das Verfahren umfassend:

   1) Durchführen einer cfDNA-Sequenzierung an einer Plasmaprobe einer schwangeren Frau, an der bekannte mütterliche und väterliche Haplotypen zu ermitteln sind, um Sequenzierungs-Reads zu erhalten;
   2) Abgleichen der Sequenzierungs-Reads mit einem Referenzgenom und das Bestimmen der Depth-Verteilung und der Mutationen der Probe anhand der Abgleichergebnisse;
   3) Berechnen der fetalen cfDNA-Konzentration entsprechend der Minor-Allel-Häufigkeit für vier Arten von Allelloci, wobei die vier Arten von Allelloci Folgendes umfassen: einen Allellocus, bei dem die Mutter homozygot und der Fötus homozygot ist, einen Allellocus, bei dem die Mutter homozygot und der Fötus heterozygot ist, einen Allellocus, bei dem die Mutter heterozygot und der Fötus homozygot ist, und einen Allellocus, bei dem die Mutter heterozygot und der Fötus heterozygot ist; und
   4) für die elterlichen Genotypkombinationen AAAB, ABAA und ABAB, Schließen auf die SNP- und InDel-Genotypen des Fötus wie folgt:

      a) für die elterliche Genotypkombination AAAB, Verwenden eines Bayes'schen Algorithmus zum Schließen auf den SNP-Genotyp des Fötus und Verwenden der CIVA-Methode zum Schließen auf die vom Vater vererbten InDel-Allele des Fötus auf der Grundlage des vom Vater vererbten SNP-Haplotyps;
      b) für die elterliche Genotypkombination ABAA, Verwenden des SPRT-Algorithmus zum Schließen auf einen Locus, der durch den Haplotyp abgedeckt werden kann, und Verwenden der CIVA-Methode zum Schließen auf die verbleibenden Loci;
      c) für die elterliche Genotypkombination ABAB, zunächst Verwenden der CIVA-Methode zur Bestimmung des vom Vater vererbten fetalen Locustyps; anschließend Verwenden des SPRT-Algorithmus zum Schließen auf den von der Mutter vererbten fetalen Lokustyp; für den Lokus, der nicht durch den SPRT-Algorithmus abgeleitet werden kann, Verwenden der CIVA-Methode zum Schließen auf den von der Mutter vererbten fetalen Alleltyp; Kombination des väterlichen Allels und des mütterlichen Allels, um den fetalen Genotyp an diesem Locus zu erhalten;
      d) für einen Locus, der durch die Haplotypdaten in den oben genannten drei Genotypkombinationen nicht abgedeckt werden kann, Verwenden des Bayes'schen Algorithmus zur Bestimmung der Genotypen des Locus; und
      e) in dem Fall, dass der durch den Bayes'schen Algorithmus abgeleitete fetale Locus sich vom elterlichen Locus unterscheidet, wird eine fetale *de novo-Mutation* identifiziert.

2. Verfahren nach Anspruch 1, wobei in 4)

   der Bayes'sche Algorithmus umfasst: Erstellen eines Bayes'schen Modells auf der Grundlage der fetalen Konzentration, um den fetalen Genotyp abzuleiten;
   der SPRT-Algorithmus umfasst: Identifizieren des fetalen Genotyps an einem Locus, an dem die Mutter heterozygot und der Vater homozygot ist, unter Verwendung des Ungleichgewichts der Read-Deckung zweier Allele an heterozygoten Loci in den Plasma-cfDNA-Sequenzierungsdaten der schwangeren Frau; und
   das CIVA-Verfahren umfasst: Ableiten von zwei Haplotypen zweier Allele, die vom Vater bzw. von der Mutter vererbt wurden, an dem vorherzusagenen fetalen Locus, und das Extrahieren der Allele, die tatsächlich an dem Locus in den beiden Haplotypen vorhanden sind, um auf den fetalen Genotyp zu schließen.

3. Verfahren nach Anspruch 1 oder 2, wobei in Schritt 4) der Bayes'sche Algorithmus umfasst: Für jeden Locus wird, wenn die Read-Tiefe verschiedener Allele und die fetale Konzentration im Plasma der Schwangeren bekannt sind, die Wahrscheinlichkeit, eine Kombination i aus mütterlichen und fetalen Genotypen zu erhalten, wie folgt berechnet:

$$P(A_i|B) = \frac{P(B|A_i)P(A_i)}{\sum_{i=1}^{n} P(B|A_i)P(A_i)}$$

wobei P($A_i$) die A-priori-Wahrscheinlichkeit der Kombination *i* unter *n* Kombinationen von mütterlichen und fetalen Genotypen ist, die auf der Grundlage der fetalen Konzentration erhalten wurden; P(B|$A_i$) die kumulative Wahrscheinlichkeit der Kombination *i* von mütterlichen und fetalen Genotypen ist, die auf der Grundlage der Read-Tiefe am Locus und der A-priori-Wahrscheinlichkeit berechnet wurde; *n* Kombinationen von mütterlichen und fetalen Genotypen = (10 mütterliche Genotypen × 10 fetale Genotypen); wenn die väterlichen und mütterlichen Genotypen bekannt sind, wird die Kombination bei der Berechnung der A-priori-Wahrscheinlichkeit für den oben genannten Bayes'schen Algorithmus modifiziert, um die Kombinationen von mütterlichen und fetalen Genotypen zu entfernen, die nicht den Mendelschen Vererbungsgesetzen entsprechen, und der Wahrscheinlichkeitswert jeder Kombination wird neu berechnet; und schließlich wird die Kombination mit der höchsten Wahrscheinlichkeit ausgewählt, um den fetalen Genotyp zu erhalten.

4. Verfahren nach Anspruch 1 oder 2, wobei im SPRT-Algorithmus alle SNPs im mütterlichen Genom als $\alpha$-Typ und $\beta$-Typ klassifiziert werden und zwei Haplotypen der Mutter als Hap I und Hap II nummeriert werden; wobei der $\alpha$-Typ-SNP so definiert ist, dass das Allel des Vaters in dem SNP mit dem der Mutter in Hap I übereinstimmt, und der $\beta$-Typ-SNP so definiert ist, dass das Allel des Vaters in dem SNP mit dem der Mutter in Hap II übereinstimmt; für den $\alpha$-Typ-SNP steigt die Anzahl der Allel-Reads an dem Locus in Hap I, wenn der Fötus Hap I von der Mutter erbt; die Anzahl der Reads der beiden Allele an diesem Locus ist jedoch nahezu ausgeglichen, wenn der Fötus Hap II von der Mutter erbt; beim SNP vom $\beta$-Typ ist die Anzahl der Reads der beiden Allele an diesem Locus nahezu ausgeglichen, wenn der Fötus Hap I von der Mutter erbt; die Anzahl der Allel-Reads an diesem Locus in Hap II steigt jedoch an, wenn der Fötus Hap II von der Mutter erbt.

5. Verfahren nach Anspruch 4, wobei im SPRT-Algorithmus die folgenden beiden Schwellenwerte verwendet werden, um zu bestimmen, ob zwei Allele an jedem Locus im Gleichgewicht sind,

$$Obergrenze = \frac{(ln\ 1200)/N - ln\ d}{ln\ g}$$

und

$$Untergrenze = \frac{(ln\ 1/1200)/N - ln\ d}{ln\ g}$$

wobei,

$$d = \frac{1 - q_1}{1 - q_0}$$

und

$$g = \frac{q_1(1 - q_0)}{q_0(1 - q_1)}$$

wobei $q_0$ die Anzahl der Reads von Hap I an diesem Locus ist, wenn der Fötus Hap II von der Mutter erbt, $q_1$ die Anzahl der Reads von Hap I an diesem Locus ist, wenn der Fötus Hap I von der Mutter erbt, und N die Gesamtzahl der Reads ist;
für den $\alpha$-Typ-SNP gilt $q_0$=0,5, $q_1$=ff/2+0,5; für den SNP vom $\beta$-Typ gilt: $q_1$=0,5, $q_0$=0,5-ff/2.

6. Verfahren nach Anspruch 1 oder 2, wobei in e) von 4) eine *de novo-Mutation* beim Fötus anhand der folgenden Kriterien weiter untersucht wird:

i) alle Varianten, die in dbSNP147, der Genome Aggregation Database (gnomAD) mit einer Minor-Allel-Häufigkeit (MAF) > 1 % in der ostasiatischen Population, sowie in der internen Datenbank erfasst sind;

ii) ein Locus mit einer Genotyp-Qualität (GQ) des väterlichen, mütterlichen oder fetalen Genotyps < 30;

iii) ein Locus mit einer Sequenzierungstiefe des väterlichen, mütterlichen oder fetalen Genotyps < 20;

iv) ein Locus mit einer Genotypplausibilität (PL) des väterlichen, mütterlichen oder fetalen Genotyps < 30;

v) ein Locus mit einer Minor-Allel-Häufigkeit des väterlichen, mütterlichen oder fetalen Genotyps < 0,3 oder > 0,7; und

vi) ein Locus mit den folgenden Qualitätskontrollkriterien in der kombinierten VCF-Datei (Variant Call Format, generiert durch GATK) von Elternteil und Fötus: QualByDepth (QD) < 2,0, RMSMappingQuality (MQ) < 50,0, FisherStrand (FS) > 60,0, StrandOddsRatio (SOR) > 3,0, MappingQualityRankSumTest (MQRankSum) < -12,5, ReadPosRankSumTest (ReadPosRankSum) < -8,0.

7. Verfahren nach Anspruch 1 oder 2, wobei in 1) die mütterlichen und väterlichen Haplotypen durch stLFR-Sequenzierung (Single Tube Long Fragment Read) der mütterlichen bzw. väterlichen Blutzellen erhalten werden.

8. Verfahren nach Anspruch 1 oder 2, wobei in 1) die cfDNA-Sequenzierungstiefe 70-200× beträgt.

9. Verfahren nach Anspruch 8, wobei die cfDNA-Sequenzierungstiefe 100× beträgt.

## Revendications

1. Méthode de détection du génotype fœtal basée sur les haplotypes, ladite méthode comprenant :

1) réaliser un séquençage de l'ADN libre circulant (cfDNA) sur un échantillon de plasma d'une femme enceinte à analyser, dont les haplotypes maternels et paternels sont connus, afin d'obtenir des lectures de séquençage ;

2) aligner les lectures de séquençage sur un génome de référence, et déterminer la distribution de la profondeur de séquençage et les mutations de l'échantillon en fonction des résultats de l'alignement ;

3) calculer la concentration en ADN libre fœtal (cfDNA) en fonction de la fréquence des allèles mineurs pour quatre types de loci alléliques, les quatre types de loci alléliques comprenant : un locus allélique où la mère est homozygote et le fœ-tus est homozygote, un locus allélique où la mère est homozygote et le fœtus est hétérozygote, un locus allélique où la mère est hétérozygote et le fœtus est homozygote, et un locus allélique où la mère est hétérozygote et le fœtus est hétérozygote ; et

4) pour les combinaisons de génotypes parentaux AAAB, ABAA et ABAB, déduire les génotypes SNP et InDel du fœtus comme suit :

a) pour la combinaison de génotypes parentaux AAAB, utiliser un algorithme bayésien pour déduire le génotype SNP du fœtus, et utiliser la méthode CIVA pour déduire les allèles InDel du fœtus hérités du père sur la base de l'haplotype SNP hérité du père ;

b) pour la combinaison de génotypes parentaux ABAA, utiliser l'algorithme SPRT pour déduire un locus pouvant être couvert par l'haplotype et utiliser la méthode CIVA pour déduire les loci restants ;

c) pour la combinaison de génotypes parentaux ABAB, utiliser d'abord la méthode CIVA pour déterminer le type de locus fœtal hérité du père ; ensuite, utiliser l'algorithme SPRT pour déduire le type de locus fœtal hérité de la mère ; pour le locus qui ne peut être déduit par l'algorithme SPRT, utiliser la méthode CIVA pour déduire le type d'allèle fœtal hérité de la mère ; combiner l'allèle paternel et l'allèle maternel, de manière à obtenir le génotype fœtal au niveau du locus ;

d) pour un locus qui ne peut pas être couvert par les données d'haplotype dans les trois combinaisons de génotypes ci-dessus, utiliser un algorithme bayésien pour déterminer les génotypes du locus ; et

e) dans le cas où le locus fœtal déduit par l'algorithme bayésien est différent du locus parental, une mutation *de novo* fœtale est identifiée.

2. Procédé selon la revendication 1, dans lequel, en 4),

l'algorithme bayésien comprend : construire un modèle bayésien basé sur la concentration fœtale pour déduire le génotype fœtal ;

l'algorithme SPRT comprend : identifier le génotype fœtal au niveau d'un locus où la mère est hétérozygote et le père homozygote en utilisant le déséquilibre de la couverture de lecture des deux allèles au niveau des loci hétérozygotes dans les données de séquençage de l'ADN libre circulant (cfDNA) plasmatique de la femme enceinte ; et

la méthode CIVA comprend : déduire deux haplotypes de deux allèles hérités respectivement du père et de la mère sur le locus fœtal à prédire, et extraire les allèles qui se trouvent effectivement sur le locus dans les deux haplotypes, de manière à déduire le génotype fœtal.

**3.** Procédé selon la revendication 1 ou 2, dans lequel, en 4), l'algorithme bayésien comprend : pour chaque locus, lorsque la profondeur de lecture des différents allèles et la concentration fœtale dans le plasma de la femme enceinte sont connues, la probabilité d'obtenir une combinaison *i* de génotypes maternels et fœtaux est calculée comme suit :

$$P(A_i|B) = \frac{P(B|A_i)P(A_i)}{\sum_{i=1}^{n} P(B|A_i)P(A_i)}$$

dans lequel P(A$_i$) est une probabilité a priori de la combinaison *i* parmi *n* combinaisons de génotypes maternels et fœtaux obtenues sur la base de la concentration fœtale ; P(B|A$_i$) est la probabilité cumulative de la combinaison *i* de génotypes maternels et fœtaux calculée sur la base de la profondeur de lecture au locus et de la probabilité a priori ; *n* combinaisons de génotypes maternels et fœtaux = (10 génotypes maternels $\times$ 10 génotypes fœtaux) ; lorsque les génotypes paternel et maternel sont connus, la combinaison utilisée dans le calcul de la probabilité a priori est modifiée pour l'algorithme bayésien ci-dessus, afin d'éliminer les combinaisons de génotypes maternels et fœtaux qui ne sont pas conformes aux lois de Mendel sur l'hérédité, et la valeur de probabilité de chaque combinaison est recalculée ; et enfin, la combinaison présentant la probabilité la plus élevée est sélectionnée, de manière à obtenir le génotype fœtal.

**4.** Procédé selon la revendication 1 ou 2, dans lequel, dans l'algorithme SPRT, tous les SNP du génome maternel sont classés en types a et β, et les deux haplotypes de la mère sont numérotés Hap I et Hap II ; le SNP de type a est défini comme l'allèle du père dans le SNP étant le même que celui de la mère dans Hap I, et le SNP de type β est défini comme l'allèle du père dans le SNP étant le même que celui de la mère dans Hap II ; pour le SNP de type a, le nombre de lectures d'allèles au locus dans Hap I augmente lorsque le fœtus hérite de Hap I de la mère ; cependant, les nombres de lectures des deux allèles au locus sont proches de l'équilibre lorsque le fœtus hérite de l'haplotype II de la mère ; pour le SNP de type β, les nombres de lectures des deux allèles au locus sont proches de l'équilibre lorsque le fœtus hérite de l'haplotype I de la mère ; cependant, le nombre de lectures d'allèles au locus dans l'haplotype II augmente lorsque le fœtus hérite de l'haplotype II de la mère.

**5.** Procédé selon la revendication 4, dans lequel, dans l'algorithme SPRT, les deux seuils suivants sont utilisés pour déterminer si les deux allèles au niveau de chaque locus sont équilibrés,

$$limite\ supérieure = \frac{(ln\ 1200)/N - ln\ d}{ln\ g}$$

et

$$limite\ inférieure = \frac{(ln\ 1/1200)/N - ln\ d}{ln\ g}$$

dans lequel,

$$d = \frac{1 - q_1}{1 - q_0}$$

et

$$g = \frac{q_1(1 - q_0)}{q_0(1 - q_1)}$$

dans lequel $q_0$ est le nombre de lectures de l'allèle I au locus lorsque le fœtus hérite de l'allèle II de la mère, $q_1$ est le nombre de lectures de l'allèle I au locus lorsque le fœtus hérite de l'allèle I de la mère, et N est le nombre total de lectures ;

pour le SNP de type $\alpha$, $q_0$=0,5, $q_1$=ff/2+0,5 ; pour le SNP de type $\beta$, $q_1$=0,5, $q_0$=0,5-ff/2.

6. Procédé selon la revendication 1 ou 2, dans lequel, en e) du point 4), une mutation *de novo* chez le fœtus est en outre dépistée selon les critères suivants :

   i) toutes les variantes répertoriées dans dbSNP147, la base de données d'agrégation génomique (gnomAD) avec une fréquence allélique mineure (MAF) > 1 % dans la population d'Asie de l'Est, et la base de données interne ;
   ii) un locus dont la qualité du génotype (GQ) paternel, maternel ou fœtal est < 30 ;
   iii) un locus présentant une profondeur de séquençage paternelle, maternelle ou fœtale < 20 ;
   iv) un locus présentant une probabilité de génotype (PL) paternelle, maternelle ou fœtale < 30 ;
   v) un locus présentant une fréquence allélique mineure paternelle, maternelle ou fœtale < 0,3 ou > 0,7 ; et
   vi) un locus répondant aux critères de contrôle qualité suivants dans le fichier VCF (Variant Call Format, généré par GATK) combiné des parents et du fœtus : QualByDepth (QD) < 2,0, RMSMappingQuality (MQ) < 50,0, FisherStrand (FS) > 60,0, StrandOddsRatio (SOR) > 3,0, MappingQualityRankSumTest (MQRankSum) < - 12,5, ReadPosRankSumTest (ReadPosRankSum) < -8,0.

7. Procédé selon la revendication 1 ou 2, dans lequel, en 1), les haplotypes maternel et paternel sont obtenus par séquençage stLFR (single tube Long Fragment Read) des cellules sanguines maternelles et paternelles, respectivement.

8. Procédé selon la revendication 1 ou 2, dans lequel, en 1), la profondeur de séquençage de l'ADN libre circulant (cfDNA) est comprise entre 70 et 200×.

9. Procédé selon la revendication 8, dans lequel la profondeur de séquençage de l'ADN libre circulant est de 100×.

Fig.1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AMICUCCI, P** ; **M. GENNARELLI** ; **G. NOVELLI** ; **B. DALLAPICCOLA**. Prenatal Diagnosis of Myotonic Dystrophy Using Fetal DNA Obtained from Maternal Plasma.. *Clinical Chemistry*, 2000, vol. 46 (2), 301-302
- **BARRETT, A. N.** ; **T. C. MCDONNELL** ; **K. C. CHAN** ; **L. S. CHITTY**. Digital PCR analysis of maternal plasma for noninvasive detection of sickle cell anemia.. *Clin Chem*, 2012, vol. 58 (6), 1026-1032
- **CHAN, K. C.** ; **P. JIANG** ; **K. SUN** ; **Y K. CHENG** ; **Y. K. TONG** ; **S. H. CHENG** ; **A. I. WONG** ; **I. HUDECOVA** ; **T. Y. LEUNG** ; **R. W. CHIU**. Second generation noninvasive fetal genome analysis reveals de novo mutations, single-base parental inheritance, and preferred DNA ends. *Proc Natl Acad Sci U S A*, 2016, vol. 113 (50), E8159-E8168
- **FAN, H. C** ; **W. GU** ; **J. WANG** ; **Y. J. BLUMENFELD** ; **Y. Y. EL-SAYED** ; **S. R. QUAKE**. Non-invasive prenatal measurement of the fetal genome.. *Nature*, 2012, vol. 487 (7407), 320-324
- **KITZMAN, J. O** ; **M. W. SNYDER** ; **M. VENTURA** ; **A. P. LEWIS** ; **R. QIU** ; **L. E. SIMMONS** ; **H. S. GAMMILL** ; **C. E. RUBENS** ; **D. A. SANTILLAN** ; **J. C. MURRAY**. Noninvasive whole-genome sequencing of a human fetus. *Sci Transl Med*, 2012, vol. 4 (137), 137-176

- **LO, Y. M.** ; **K. C. CHAN** ; **H. SUN** ; **E.Z. CHEN** ; **P. JIANG** ; **F. M. LUN** ; **Y. W. ZHENG** ; **T. Y. LEUNG** ; **T. K. LAU** ; **C. R. CANTOR**. Maternal plasma DNA sequencing reveals the genome-wide genetic and mutational profile of the fetus.. *Sci Transl Med*, 2010, vol. 2 (61), 61-91
- **RABINOWITZ, T** ; **A. POLSKY** ; **D. GOLAN** ; **A. DANILEVSKY** ; **G. SHAPIRA** ; **C. RAFF** ; **L. BASEL-SALMON** ; **R. T. MATAR** ; **N. SHOMRON**. Bayesian-based noninvasive prenatal diagnosis of single-gene disorders. *Genome Res*, 2019, vol. 29 (3), 428-438
- **SAITO, H** ; **A. SEKIZAWA** ; **T. MORIMOTO** ; **M. SUZUKI** ; **T. YANAIHARA**. Prenatal DNA diagnosis of a single-gene disorder from maternal plasma. *Lancet*, 2000, vol. 356 (9236), 1170
- **TSUI, N. B. Y** ; **R. A. KADIR** ; **K. C. A. CHAN** ; **C. CHI** ; **Y. M. D. LO**. Noninvasive prenatal diagnosis of hemophilia by microfluidics digital PCR analysis of maternal plasma DNA.. *Blood*, 2011, vol. 117 (13), 3684-3691